# EUROPÄISCHE PATENTSCHRIFT

(11) **EP 3 530 312 B1**
(45) Veröffentlichungstag und Bekanntmachung des Hinweises auf die Patenterteilung: **10.04.2024**
(21) Anmeldenummer: 19157634.7
(22) Anmeldetag: 18.02.2019
(51) Int. Cl.: A61M 39/10, A61M 39/16, A61M 39/12

(54) **NICHT ENTKONNEKTIERBAR SICHERBARER KONNEKTOR MIT ÜBERWURFHÜLSE**
SECURABLE CONNECTOR WITH SLEEVE WHICH CANNOT BE DISCONNECTED
CONNECTEUR POUVANT ÊTRE BLOQUÉ NE POUVANT PAS ÊTRE DÉCONNECTÉ POURVU DE MANCHON D'ACCOUPLEMENT

(30) Priorität: 21.02.2018 DE 102018103902
(43) Veröffentlichungstag der Anmeldung: 28.08.2019
(73) Patentinhaber: CODAN Medizinische Geräte GmbH, 23738 Lensahn (DE)
(72) Erfinder: Crombach, Jozef Jacobus Maria, 7711 VR Nieuwleusen (NL)
(74) Vertreter: Raffay & Fleck

(56) Entgegenhaltungen:
- EP-A1- 3 081 253
- US-A1- 2003 201 639
- US-A1- 2008 172 039

## Beschreibung

Diese Erfindung betrifft einen Konnektor, insbesondere für die Verwendung in Zuleitungen intravenöser Infusionen, insbesondere ein Luer-Lock-System, welches mit einem Gegenstück verbunden werden kann, wobei der Konnektor eine Überwurfhülse aufweist, die auf dem Konnektor verrastet werden kann und dadurch ein Entkonnektieren des Konnektors verhindert/erschwert.

Konnektoren zum Verbinden verschiedener Teilstücke eines Systems sind seit langem, bspw. aus der US 2008/0172039 A1 oder US 2003/0201639 A1, bekannt. Dabei ist besonders eine formschlüssige Verbindung beider Teilstücke notwendig, um einen Transport einer Substanz durch das gesamte System ohne Verlust der Substanz und unter Verhinderung des Eindringens von Fremdstoffen an der Verbindung zu ermöglichen. Insbesondere beinhaltet ein solches System ein Anschlussstück und ein Gegenstück, insbesondere ein positives Anschlussstück und als Gegenstück ein negatives Anschlussstück. In einem medizinischen System wird besonders vorteilhafterweise ein Luer-Lock System verwendet, welches den Transport einer Flüssigkeit ohne Verlust an einer Verbindung ermöglicht. Dabei beinhaltet das Anschlussstück einen Konus und, im Luer-Lock System, ein Gewinde, welches zum einen eine formschlüssige und dichte Verbindung sowie eine sichere Verbindung gewährleistet. Besonders im Bereich der Medizintechnik sind seit langem unterschiedlichste Konnektoren bekannt wobei sich das Luer-Lock System durchgesetzt hat und überwiegend Anwendung findet. Durch die ISO Norm 80369-7 und die DIN EN 20594 sind die Formen des Konnektors definiert und somit Systeme unterschiedlicher Hersteller miteinander kompatibel und kombinierbar. Das Luer System erlaubt es, das Anschlussstück und das Gegenstück, insbesondere das positive Anschlussstück das negative Anschlussstück, insbesondere das männliche Anschlussstück und das weibliche Gegenstück, eines Luer Systems einfach miteinander zu verbinden. Dabei können das Anschlussstück und das Gegenstück Gewinde aufweisen, wodurch das Anschlussstück und das Gegenstück ineinander und miteinander verschraubt werden können. Dies ermöglicht eine Verbindung beider Teilstücke, so dass das Anschlussstück und das Gegenstück nicht versehendlich wieder voneinander getrennt werden kann. Ein solches wird als Luer-Lock System bezeichnet. Ein Nachteil eines solchen Systems liegt darin, dass es wieder lösbar ist. Ein solches Lösen kann zum einen absichtlich oder unabsichtlich im Laufe der weiteren Handhabung während oder in Vorbereitung des Systems, insbesondere einer medizinischen Behandlung, geschehen.

Um ein Entkonnektieren zuverlässig zu vermeiden, sind im Stand der Technik verschiedene Sicherungsmechanismen bekannt. Unter anderem offenbart die EP 1917996 B1 ein Luer Lock System, dass das Drehen des Gewindes nur in die Richtung zum Verschrauben ermöglicht und damit gegen ein Entkonnektieren sichert. Sobald eine entsprechende Verbindung durch die Verschraubung gesichert ist, kann die Verschraubung per Hand nicht mehr gelöst werden. Dies wird dadurch erreicht, dass ein um das Anschlussstück angeordnete Verzahnungsmittel aufweist und das um das Anschlussstück drehbare Lock Gewinde ein dazu passgerechtes Gegenverzahnungsmittel aufweist, wodurch in einem verrasten Zustand die Überwurfhülse das Anschlussstück mit dem Gegenstück mittels der Verzahnung innerhalb des Gewindes unlösbar miteinander verbindet. Dies hat den Nachteil, dass, sobald die Infusionsanordnung aufgebaut ist, dieses System unlösbar verrastet ist, und somit ein nachträgliches Entkonnektieren nicht mehr möglich ist.

Ein Grund, der ein Lösen der Verbindung erforderlich macht, mag in einem Fehler des Aufbaus des Systems, wie z. B. einem fehlerhaften Aufbau eines Infusionsbaums liegen. Dies erfordert bei Verwendung bekannter nicht entkonnektierbarer Konnektoren ein Verwerfen des fehlerhaften, insbesondere teuren und/oder einzigen, Aufbaus.

Demgegenüber bietet das klassische Luer System (ohne Lock) ein Korrigieren aber keine Sicherung gegen Entkonnektieren. Eine solche Lösung beschreibt auch das Gebrauchsmuster DE 20109061 U1. Diesem liegt die Aufgabe zu Grunde eine Verbindung bereitzustellen, die mit geringem Kraftaufwand wieder gelöst werden kann. Dabei weist ein solches System sowohl ein Anschlussstück zum Anschluss an ein Gegenstück, sowie eine Kupplung auf, wobei diese ausgestattet mit einer Überlastkupplung auf das Anschlussstück gedreht werden kann und bei Erreichen eines Grenzwertes des Drehmoments die Hülse mithilfe eines Griffteils weiter gedreht werden kann, jedoch das Anschlussstück nicht weiter in einer Drehung mitnimmt. Dadurch kann ein zu festes Anziehen der Verbindung vermieden werden. Dabei kann diese Verbindung durch ein Drehen in entgegengesetzte Richtung wieder gelöst werden.

Um ein sicheres und fluiddichtes Halten eines Schlauches an einem Konnektor zu gewährleisten, wurde z. B. in der EP 3081253 A1 vorgeschlagen, an einem Endabschnitt des Konnektors Vorsprünge anzubringen, mit deren Hilfe und einer Mutter der Schlauch zwischen Konnektor, Endabschnitt des Konnektors, und Mutter derart geklemmt wird, dass dadurch ein sicherer Halt des Schlauchs am Konnektor entsteht.

Dieser Erfindung liegt die Aufgabe zu Grunde, einen Konnektor anzugeben, insbesondere ein Luer System, insbesondere Konnektor eines Luer Lock Systems, der derart ausgestaltet ist, dass ein Aufbau der Infusionsanordnung, insbesondere eines Infusionsbaums, unter Verbindung mindestens eines Anschlussstücks mit einem Gegenstück, ermöglicht wird und dass das Anschlussstück mit dem Gegenstück zu einem späteren Zeitpunkt nicht-entkonnektierbar und unlösbar miteinander verbunden bzw. verschlossen werden kann. Dabei besteht zudem die Aufgabe darin, dass nach dem Aufbau die Infusionsanordnung kontrolliert werden kann, die Infusionsanordnung wieder demontiert werden könnte, die Infusionsanordnung aber bereits gegen versehentliches Lösen gesichert ist, und dass das Überführen der Infusionsanordnung in einen nicht-entkonnektierbar Zustand nicht unabsichtlich erfolgen kann. Aufgabe ist auch die Angabe eines entsprechenden Systems und eines Verfahrens.

Diese Aufgabe wird gelöst durch einen Konnektor gemäß Anspruch 1, einem System mit mehreren solcher Konnektoren gemäß Anspruch 12, sowie durch ein Verfahren zur Erstellung einer Infusionsanordnung, insbesondere eines Infusionsbaumes, gemäß Anspruch 13. Vorteilhafte Aus- und Weiterbildungen ergeben sich aus den abhängigen Ansprüchen.

Erfindungsgemäß wird die Aufgabe durch einen Konnektor und/oder Adapter, insbesondere ein Luer System, insbesondere einem Konnektor eines Luer Lock Systems, gelöst, wobei der Konnektor ein Anschlussstück, insbesondere ein positives Anschlussstück, welches mit einem passenden Gegenstück, insbesondere einem negativen Gegenstück, insbesondere einem normgerechten negativen Luer Lock Gegenstück, zum Zweck des Transports von Substanzen, insbesondere Flüssigkeiten, verbunden werden kann. Dabei weist das Anschlussstück einen Konus, insbesondere einen Luer Konus, und ein Sicherungsmittel, insbesondere ein Gewinde, insbesondere ein Lock Gewinde, insbesondere ein zumindest teilweise den Konus umschließendes Sicherungsmittel, insbesondere Gewinde, zum reversiblen Sichern einer Verbindung von Anschlussstück und Gegenstück, insbesondere unter Zusammenwirken mit mindestens einem passenden und/oder komplementären Gegensicherungsmittel des Gegenstücks, beispielsweise ein Gewinde, insbesondere gegen versehendliches Lösen der Verbindung, auf. Des Weiteren weist das Anschlussstück einen, insbesondere endständigen, insbesondere am vom Konus abgewandten Ende, und/oder mit dem Konus zum Transport von Substanzen, insbesondere Flüssigkeiten, verbundenen, Schlauch auf. Zusätzlich weist der Konnektor einen Verschiebebereich auf, wobei insbesondere der Verschiebebereich den Schlauch oder einen Abschnitt des Schlauchs und/oder zumindest einen Abschnitt des Anschlussstücks zumindest teilweise beinhaltet und/oder der Verschiebebereich auf dem Schlauch und/oder zumindest einem Abschnitt des Anschlussstücks ausgebildet ist. Der Konnektor weist zudem eine Überwurfhülse auf, wobei der Konnektor so ausgebildet ist, dass die Überwurfhülse, insbesondere auf dem Verschiebebereich, insbesondere auf dem Schlauch, verschiebbar angeordnet ist und/oder so ausgebildet ist, dass die Überwurfhülse zumindest teilweise um das Anschlussstück und/oder den Schlauch und/oder den Verschiebebereich und/oder den Schlauch, insbesondere um das Sicherungsmittel, und/oder auf dem Verschiebebereich drehbar angeordnet ist, wobei die Drehbarkeit insbesondere um die Längserstreckung des Verschiebebereichs und/oder um die Symmetrieachse und/oder Längserstreckung des Konus, insbesondere des Luer-Konus, gegeben ist. An dem Konnektor, insbesondere an dem Anschlussstück, insbesondere an einem dem Konus gegenüberliegenden Endabschnitts des Anschlussstücks und/oder einem dem Konus zugewandten Endabschnitts des Verschiebebereichs, und/oder an der Überwurfhülse ist zudem mindestens ein Rastmittel vorgesehen, um ein unlösbares Verrasten des Anschlussstücks mit der Überwurfhülse zu ermöglichen, wobei der Konnektor so ausgebildet ist, dass in einem unumkehrbar verrasteten Zustand des Konnektors, insbesondere des Anschlussstücks mit der Überwurfhülse, ein Verschieben der Überwurfhülse relativ zum Anschlussstück in alle Richtungen begrenzt ist.

Insbesondere ist an dem Anschlussstück und an der Überwurfhülse jeweils mindestens ein Rastmittel vorgesehen, wobei die Rastmittel so ausgebildet sind, dass die Rastmittel des Anschlussstücks mit den Rastmitteln der Überwurfhülse so zusammenwirken können, dass sie das Verrasten des Anschlussstücks mit der Überwurfhülse bewirken können. Insbesondere ist das mindestens eine Rastmittel so ausgebildet, dass ein Verschieben der Überwurfhülse in Richtung des Konus über eine vorgegebene Position hinaus, insbesondere ein Verschieben zu einem konusseitigen Ende des Verschiebebereichs, das Verrasten bewirkt.

Die Überwurfhülse und der Verschiebebereich sind derart ausgebildet, dass die Überwurfhülse mindestens ein Eingriffsmittel aufweist, wobei das mindestens eine Eingriffsmittel so eingerichtet ist, mit dem Verschiebebereich zumindest teilweise verbunden zu sein, insbesondere kraftschlüssig verbunden zu sein, und/oder so eingerichtet ist, den Verschiebebereich zumindest teilweise zu umschlie-ßen, insbesondere von mindestens zwei Seiten. Hierfür ist das mindestens eine Eingriffsmittel derart ausgebildet, dass das mindestens eine Eingriffsmittel eine Öffnung definiert, die kleiner ist als eine maximale Erstreckung des Querschnitts durch einen Abschnitt des oder den gesamten Verschiebebereich(s) senkrecht zu einer Längsterstreckung des Verschiebebereichs. Hierdurch wird erreicht, dass, dass zwischen dem mindestens einem Eingriffsmittel und dem Verschiebebereich ein Widerstand besteht, der einer Verschiebung der Überwurfhülse relativ zum Anschlussstück entgegen wirkt, sodass die Kraft, die benötigt wird, um die Überwurfhülse, insbesondere die Eingriffsmittel und/oder die Rastmittel auf dem Verschiebebereich, insbesondere bezüglich ihrer relativen Position auf dem Verschiebebereich, insbesondere entlang des Verschiebebereichs, zu verschieben, größer ist als die Gewichtskraft der Überwurfhülse, insbesondere beträgt die Kraft mindestens das 1,1 fache der Gewichtskraft der Überwurfhülse, insbesondere ist sie größer als ein Vielfaches der Gewichtskraft der Überwurfhülse. Alternativ oder zusätzlich wird damit erreicht, dass die Eingriffsmittel eine Öffnungsweite bilden, die so ausgewählt ist, dass die Eingriffsmittel auf einem Abschnitt des oder dem gesamten Verschiebebereich unter Ausübung einer den Verschiebebereich komprimierenden Kraft mit einem Untermaß auf den Umfang des Verschiebebereichs anliegen. Alternativ oder zusätzlich wird damit erreicht, dass die Überwurfhülse auf dem Verschiebebereich mittels einer parallel zur Längserstreckung des Verschiebebereichs und/oder der Überwurfhülse, insbesondere zusätzlich zur Gewichtskraft der Überwurfhülse, wirkenden Kraft von weniger als 5 N und/oder mehr als 0,01 N, insbesondere weniger als 1 N, insbesondere mehr als 0,1 N, insbesondere mit einer Kraft zwischen 0,1 und 0,9 N, insbesondere zwischen 0,12 und 0,81 N, verschiebbar ist.

Durch derartige Ausführungen der Überwurfhülse und/oder des Verschiebebereichs wird bewirkt, dass die Überwurfhülse aus einer Ruhelage auf dem Verschiebebereich nicht ohne weitere Einwirkung, wie zum Beispiel einer zusätzlichen äußeren Kraft, bewegt wird. Somit lässt sich ein Infusionssystem aufbauen bzw. der Konnektor mit einem Gegenstück verbinden und durch die Sicherungsmittel sichern, ohne Gefahr zu laufen, die Überwurfhülsen unabsichtlich zu verschieben und/oder zu verrasten. Somit sind Korrekturen möglich, bis durch das bewusste Verrasten der Überwurfhülse ein nicht-entkonnektierbarer Zustand geschaffen wird.

Die Überwurfhülse und/oder das Anschlussstück, der Konnektor und/oder das Gegenstück ist/sind so ausgebildet,
dass im unlösbar verrasteten Zustand des Anschlussstücks (2) mit der Überwurfhülse das Bedienen und/oder Lösen des Sicherungsmittels durch zumindest teilweises Verdecken des Sicherungsmittels und/oder des Gegenstückes und/oder des Konnektors erschwert und/oder verhindert wird, insbesondere durch Verdecken durch die Überwurfhülse (11). Dies erfolgt vorteilhafterweise dadurch, dass in einem unlösbar verrasteten Zustand der Überwurfhülse mit dem Anschlussstück, insbesondere mittels der Rastmittel, zumindest einen Abschnitt des Anschlussstücks, das Sicherungsmittel und alle damit steif verbundenen Teile, insbesondere das Anschlussstück und/oder das Sicherungsmittel und/oder das Gegenstück, insbesondere Gegensicherungsmittel des Gegenstücks, zumindest teilweise oder vollständig oder alle deren zugänglichen Bereiche umschlossen und/oder verdeckt werden, insbesondere durch die Überwurfhülse, und/oder dass in einem unlösbar verrasteten Zustand des Anschlussstücks mit der Überwurfhülse der Zugriff auf zum Lösen des Sicherungsmittels und/oder Gegensicherungsmittels und/oder zum Reversieren der Sicherung, insbesondere zwischen Anschlussstück und Gegenstück, benutzbare Abschnitte des Konnektors, insbesondere des Sicherungsmittels, und/oder Gegenstücks, insbesondere Gegensicherungsmittels, insbesondere des positiven Anschlussstücks, und/oder des Gegenstücks, zumindest teilweise oder vollständig oder alle deren zugänglichen Bereiche umschlossen und/oder verdeckt werden, insbesondere durch die Überwurfhülse, und/oder
dass in einem unlösbar verrasteten Zustand des Anschlussstücks mit der Überwurfhülse das Bedienen und/oder das Lösen des Sicherungsmittels und/oder Gegensicherungsmittels und/oder Reversieren der Sicherung durch zumindest teilweises oder vollständiges Verdecken und/oder Umschließen des Sicherungsmittels und/oder Anschlussstücks und/oder Gegensicherungsmittels und/oder des Gegenstücks und/oder des Konnektors und/oder alle deren zugänglichen Bereiche erschwert und/oder verhindert, insbesondere durch ein Verdecken und/oder Umschließen durch die Überwurfhülse.

Vorteilhafterweise ist der Konnektor so ausgebildet, dass zumindest im unlösbar verrasteten und/oder im nicht unlösbar verrasteten Zustand der Überwurfhülse mit dem Anschlussstück die Überwurfhülse um den Konnektor, insbesondere um das Anschlussstück und/oder das Sicherungsmittel und/oder den Schlauch und/oder den Verschiebebereich, insbesondere in alle Drehrichtungen um eine Drehachse, drehbar ist, wobei insbesondere das zum Drehen benötigte Drehmoment geringer ist als 0,08 Nm, insbesondere kleiner als 0,01 Nm, insbesondere die Überwurfhülse frei drehbar ist.

Vorteilhafterweise weist der Verschiebebereich eine Längserstreckung, insbesondere eine Längserstreckung parallel zur Längserstreckung des Anschlussstücks und/oder der Überwurfhülse, von mindestens 1 cm, insbesondere mindestens 5 cm, insbesondere mindestens 10 cm, auf.

Vorteilhafterweise ist das Eingriffsmittel der Überwurfhülse so eingerichtet, dass der einer Verschiebung der Überwurfhülse relativ zum Anschlussstück entgegen wirkende Widerstand auf einer Länge von mindestens 1 cm, insbesondere mindestens 5 cm, insbesondere mindestens 10 cm, auf dem Verschiebebereich und/oder auf mindestens 10%, insbesondere mindestens 50%, der Länge des Verschiebebereichs besteht.

Die Überwurfhülse ist insbesondere in zwei einander entgegengesetzte Drehrichtungen um eine Drehachse drehbar, wobei die Drehachse insbesondere parallel zur Längserstreckung des Verschiebebereichs und/oder der Überwurfhülse und/oder des Konnektors und/oder Längserstreckung des Konus, insbesondere des Luer-Konus, liegt. Dabei stellt die Drehachse insbesondere zumindest für einen Abschnitt des Konnektors und/oder den Schlauch eine Rotationssymmetrieachse dar.

Vorteilhafterweise ist das mindestens eine Rastmittel so eingerichtet, dass das unumkehrbare und/oder unlösbare Verrasten des Rastmittels eine Kraft von mindestens 27,5 N und/oder maximal 60 N, insbesondere mindestens 35 N und/oder maximal 55 N, insbesondere mindestens 30 N und/oder maximal 51 N, erfordert.

Vorteilhafterweise ist der Konnektor im unlösbar verrasteten Zustand des Konnektors nur durch zumindest teilweises Zerstören und/oder teilweises Verformen des Konnektors und/oder Gegenstücks, insbesondere der Überwurfhülse, insbesondere teilweises Zerstören der Überwurfhülse und/oder des Anschlussstücks, lösbar.

Vorteilhafterweise wird das Lösen und/oder Reversieren der revisiblen Sicherung, insbesondere ein Lösen einer Verschraubung, zwischen Konnektor, insbesondere dem Anschlussstück und dem Gegenstück, im unlösbar verrasteten Zustand dadurch erschwert, insbesondere dadurch verhindert, dass die Überwurfhülse im unlösbar verrasteten Zustand zumindest teilweise oder vollständig das Sicherungsmittel und/oder Gegensicherungsmittel und insbesondere alle damit drehsteif verbunden Teile und/oder den Konnektor und/oder das Gegenstück, insbesondere alle damit drehsteif verbunden Teile, umschließt.

Vorteilhafterweise weist die durch die Eingriffsmittel der Überwurfhülse des Konnektors gebildete Öffnungsweite einen kleineren Durchmesser als der maximale äußere Durchmesser des Verschiebebereichs und/oder des oder eines Abschnitts des Verschiebebereichs auf. Besonders vorteilhaft weist eine solche Öffnungsweite einen um mindestens 0,05 mm und/oder um maximal 0,1 mm kleineren Durchmesser als der maximale äußere Durchmesser des Verschiebebereichs und/oder des oder eines Abschnitts des Verschiebebereichs auf und/oder einen mindestens 5 % kleineren Durchmesser als der maximale äußere Durchmesser des Verschiebebereichs und/oder des oder eines eines Abschnitts des Verschiebebereichs auf.

Vorteilhafterweise weist die Überwurfhülse des Konnektors mindestens ein erstes Rastmittel auf, welches eine Rastmittelöffnungsweite ausbildet, und weist das Anschlussstück mindestens ein zweites Rastmittel auf, welches einen Rastmittelaußendurchmesser definiert, wobei der Durchmesser der Rastmittelöffnungsweite weniger als 8 mm, insbesondere mindestens 5,5 mm, insbesondere 6,1 ± 0,5 mm, beträgt und/oder kleiner, insbesondere um mindestens 2 % kleiner, ist als der Durchmesser des durch die Rastmittel des Anschlussstücks definierten Rastmittelaußendurchmessers. Zusätzlich oder alternativ definieren die Rastmittel des des Anschlussstücks mit ihren äußeren Erstreckungen einen einen Außendurchmesser, der kleiner, insbesondere um 2 % kleiner, ist als der Innendurchmesser eines Abschnitts der Überwurfhülse, in und/oder an welchem die Rastmittel des Anschlussstücks im unlösbar verrasteten Zustand des Anschlussstücks mit der Überwurfhülse aufgenommen werden und/oder anliegen, insbesondere einen Außendurchmesser von maximal 8 mm aufweist und/oder einen Außendurchmesser, der größer, insbesondere um mindestens 2 % größer, ist als der Durchmesser der durch die Eingriffsmittel gebildeten Öffnungsweite, insbesondere einen Außendurchmesser von mindestens 5,5 mm, insbesondere 6,6 ±0,1 mm.

Vorteilhafterweise ist der Konnektor und insbesondere das mindestens eine Rastmittel, sind insbesondere erste und zweite Rastmittel, so ausgebildet, dass es, insbesondere sie, unter Kraftaufwendung und reversibler Verformung des Rastmittels, insbesondere der ersten und/oder zweiten Rastmittel, in einer Bewegungsrichtung, insbesondere Entlang der Längserstreckung des Konnektors, des Anschlussstücks, des Verschiebebereichs, der Überwurfhülse und/oder des Schlauchs übereinander hinweg bewegbar sind und/oder in einer zweiten der ersten entgegengesetzten Bewegungsrichtung nur unter Zerstörung und/oder unelastischer Verformung des mindestens einen Rastmittels übereinander hinweg bewegbar sind.

Vorteilhafterweise weisen die Eingriffsmittel der Überwurfhülse des Konnektors mindestens einen Steg, insbesondere mindestens zwei gegenüberliegende Stege, insbesondere mindestens drei Stege, insbesondere mehr als drei Stege, welche Kreissegmente gleicher Breite und/oder mit gleichen Abständen zueinander ausbilden, auf.

Vorteilhafterweise weisen die Rastmittel der Überwurfhülse und/oder des Anschlussstücks, jeweils mindestens einen Steg insbesondere Raststeg, insbesondere mindestens jeweils zwei gegenüberliegende Stege, insbesondere Raststege, insbesondere jeweils mindestens drei Stege, insbesondere Raststege, welche Ringsegmente, insbesondere gleicher Breite und/oder mit gleichen Abständen zueinander, ausbilden, auf. Dabei erstrecken sich die Ringsegmente insbesondere jeweils über 10 bis 30°, insbesondere 15 bis 25°, insbesondere 20° eines Kreises um den Mittelpunkt.

Vorteilhafterweise sind die Rastmittel der Überwurfhülse und/oder des Anschlussstücks, derart ausgebildet, dass diese in ihrer Querschnittsebene parallel zur Längserstreckung des Konnektors und/oder der Überwurfhülse eine Trapezform aufweisen, wobei diese Trapezform insbesondere einen Innenwinkel von mehr als 40° und weniger als 50°, insbesondere von 45°, aufweist, wobei diese Trapezform insbesondere einen Innenwinkel senkrecht zur Längserstreckung der Überwurfhülse und parallel zur Längserstreckung der Überwurfhülse von mehr als 40° und weniger als 50°, insbesondere von 45°, aufweist.

Vorteilhafterweise bilden die Rastmittel der Überwurfhülse und/oder des Anschlussstücks, insbesondere im unlösbar verrasteten Zustand des Anschlussstücks mit der Überwurfhülse, insbesondere in einer Aufsicht auf die Rastmittel, die parallel zur Längserstreckung des Anschlussstücks und/oder der Überwurfhülse ist, insbesondere zusammen, einen fast vollständigen, insbesondere zu mindestens 90% geschlossenen, Ring aus. Insbesondere bilden die Rastmittel einen solchen Ring unabhängig von der rotativen Lage der Überwurfhülse aus, also insbesondere unabhängig von einer Drehung um die Drehachse.

Vorteilhafterweise bilden die Rastmittel des Anschlussstücks in einer Aufsicht auf die Rastmittel parallel zur Längserstreckung des Anschlussstücks und/oder der Überwurfhülse jeweils ein Segment eines Ringes aus, wobei die längste Sehne durch jedes Segment des Rastmittels des Anschlussstücks größer ist als der Abstand zwischen den Rastmitteln der Überwurfhülse, insbesondere der nebeneinanderliegenden Rastmittel der Überwurfhülse, insbesondere nebeneinanderliegender Rastmittel der Überwurfhülse, den die Rastmittel der Überwurfhülse in einer Aufsicht auf die Rastmittel parallel zur Längserstreckung des Anschlussstücks und/oder der Überwurfhülse aufweisen und/oder wobei der Kreiswinkel, über den sich Ringsegment erstreckt des Anschlussstücks größer ist als der Kreiswinkel zwischen den Rastmitteln der Überwurfhülse, insbesondere der nebeneinanderliegenden Rastmittel der Überwurfhülse, insbesondere nebeneinanderliegender Rastmittel des Anschlussstücks. Zusätzlich und/oder alternativ können die Rastmittel des der Überwurfhülse so ausgebildet sein, dass sie in einer Aufsicht auf die Rastmittel parallel zur Längserstreckung des Anschlussstücks und/oder der Überwurfhülse jeweils ein Segment eines Ringes ausbilden, wobei die längste Sehne durch jedes Segment des Rastmittels der Überwurfhülse größer ist als der Abstand zwischen den Rastmitteln des Anschlussstücks und/oder wobei der Kreiswinkel, über den sich Ringsegment erstreckt der Überwurfhülse größer ist als der Kreiswinkel zwischen den Rastmitteln des Anschlussstücks, insbesondere der nebeneinanderliegenden Rastmittel des Anschlussstücks, insbesondere der in einem Ring aufeinanderfolgenden Rastmittel des Anschlussstücks, den die Rastmittel des Anschlussstücks in einer Aufsicht auf die Rastmittel parallel zur Längserstreckung des Anschlussstücks und/oder der Überwurfhülse aufweisen. Zusätzlich und/oder alternativ können die Rastmittel des Anschlussstücks und/oder der Überwurfhülse so ausgebildet sein, dass sie in einer Aufsicht auf die Rastmittel parallel zur Längserstreckung des Anschlussstücks und/oder der Überwurfhülse jeweils ein Segment eines Ringes ausbilden, wobei ein erstes Rastmittel des Anschlussstücks zumindest teilweise von einem zweiten Rastmittel der Überwurfhülse verdeckt wird, insbesondere jedes Rastmittel des Anschlussstücks zumindest teilweise gleichzeitig von jeweils mindestens einem, insbesondere zwei, Rastmittel der Überwurfhülse verdeckt wird, wobei insbesondere eine solche Verdeckung unabhängig von der rotativen Lage der Überwurfhülse relativ zum Anschlussstück erfolgt. Zusätzlich und/oder alternativ können die Rastmittel des Anschlussstücks und der Überwurfhülse so ausgebildet sein, dass sie in einer Aufsicht auf die Rastmittel parallel zur Längserstreckung des Anschlussstücks und/oder der Überwurfhülse jeweils ein Segment eines Ringes ausbilden, wobei in dem unlösbar verrasteten Zustand des Anschlussstücks mit der Überwurfhülse in jeder rotativen Lage der Rastmittel des Anschlussstücks und der Überwurfhülse mindestens ein Rastmittel des Anschlussstücks von jeweils mindestens einem, insbesondere zwei, Rastmittel der Überwurfhülse zumindest teilweise überdeckt wird.

Durch derartige Ausbildungen der Rastmittel lässt sich eine Anordnung erreichen, die ein besonderes einfaches und Fehler unanfälliges und sicheres Verrasten ermöglicht.

Vorteilhafterweise ist das Sicherungsmittel, insbesondere das Gewinde, insbesondere das Lock Gewinde, des Anschlussstücks des Konnektors fest und/oder steif mit dem Konus, insbesondere dem Luer Konus, und/oder mit dem Anschlussstück verbunden, vorzugsweise einstückig damit ausgebildet.

Vorteilhafterweise sind die Eingriffsmittel der Überwurfhülse an der Überwurfhülse distal und/oder am von dem Sicherungsmittel entfernten Ende der Überwurfhülse ausgebildet und/oder sind die Eingriffsmittel der Überwurfhülse am vom Konus entfernten Ende des Anschlussstücks und/oder des Verschiebebereichs, insbesondere auf dem Schlauch, angeordnet.

Dadurch lässt sich eine besonders kompakte und einfach zu bedienende Ausführung erreichen.

Vorteilhafterweise weist der Konnektor ein Zwischenstück auf, welches zwischen dem Sicherungsmittel, insbesondere dem Gewinde, insbesondere dem Luer-Gewinde, und einem endständigen Abschnitt des Anschlussstücks, insbesondere dem Sicherungsmittel gegenüberliegenden Abschnittes des Anschlussstückes, ausgebildet ist, wobei der Außenumfang eines ersten Abschnittes, insbesondere eines Abschnittes parallel zur Längserstreckung des Konnektors, des Zwischenstücks teilweise einen größeren Umfang als der Außenumfang eines zweiten Abschnittes des Zwischenstücks aufweist, insbesondere einen Umfang, dessen Durchmesser größer ist als der Umfang, den der Verschiebebereich bildet, und insbesondere kleiner ist als der Durchmesser, den die Überwurfhülse an einem Abschnitt mit einem größten Innenumfang aufweist. Insbesondere ist das Zwischenstück nur teilweise in einer Längserstreckung parallel zur Längserstreckung des Konus, insbesondere des Luer-Konus, ausgebildet. Somit kann besonderes vorteilhaft die Verschraubung des Gegenstücks mit dem am Zwischenstück drehsteif verbundenen Gewinde und alle drehsteif damit verbundenen Teilen des Anschlussstückes, vereinfacht erfolgen. Vorteilhafterweise werden dabei Griffmittel, die dieses Zwischenstück ausbildet, bei dem Überwurf der Überwurfhülse und dem Verrasten der Überwurfhülse mit dem Anschlussstück verdeckt.

Insbesondere ist der Konnektor mit einem am Anschlussstück angrenzend angeordneten Schlauch, insbesondere mit einem Außendurchmesser von 3,95 bis 4,15 mm ausgebildet. Insbesondere weist die Überwurfhülse eine Läge von 20 bis 40 mm und/oder der Verschiebebereich eine Länge von mindestens 20 bis 40 mm und/oder von mindestens der Länge der Überwurfhülse auf. Insbesondere weist die Überwurfhülse einen Außendurchmesser von 8 bis 20 mm auf. Insbesondere weist das Anschlussstück eine Läge von 20 bis 40 mm und/oder weist die Überwurfhülse eine Länge von mindestens 80%, insbesondere mindestens 90%, der Länge des Anschlussstücks auf. Insbesondere sind Anschlussstück und/oder Überwurfhülse so ausgebildet, das im unlösbar verrasteten Zustand die relative Bewegung zwischen Anschlussstück und Überwurfhülse in Richtung derer Längserstreckung und/oder in Richtung der Verschiebung auf dem Verschiebebereich maximal 4, insbesondere maximal 2 mm, beträgt.

Insbesondere beträgt der Abstand zwischen Eingriffsmitteln und Rastmitteln der Überwurfhülse 0,5 bis 3 mm, insbesondere 0,8 bis 2mm.

Gelöst wird die Aufgabe auch durch ein System, welches mindestens einen erfindungsgemäßen Konnektor und mindestens ein Gegenstück aufweist, wobei das Gegenstück und/oder der Konnektor eingerichtet ist/sind, zwecks Substanztransport, insbesondere Flüssigkeitstransports, verbunden zu werden und/oder wobei das Gegenstück insbesondere ein negatives Gegenstück ist, insbesondere eine normgerechtes negatives Luer-Lock Gegenstück.

Insbesondere weist das Gegenstück ein Gegensicherungsmittel, insbesondere ein zum Gewinde passendes Gegengewinde auf. Weitere oben beschriebene vorteilhafte Ausbildungen sind auf das System übertragbar.

Die Aufgabe wird auch durch ein Verfahren zum Erstellen einer Infusionsanordnung, insbesondere zum Transport und/oder zur Verabreichung von Substanzen, insbesondere Flüssigkeiten, insbesondere eines Infusionsbaums, gelöst, wobei dies unter und/oder durch Verbinden eines Anschlussstücks, insbesondere eines erfindungsgemäßen Konnektors, mit einem Gegenstück, insbesondere unter Verwendung eines erfindungsgemäßen Systems und/oder Konnektors, erfolgt, wobei
das Anschlussstück mit dem Gegenstück reversible verbunden und diese Verbindung reversibel gesichert wird, insbesondere durch Sicherungsmittel, insbesondere durch eine reversible Verschraubung, und
das Lösen der reversiblen Sicherung durch Aufschieben, insbesondere Überwurf, einer Überwurfhülse und unlösbares Verrasten der Überwurfhülse mit dem Anschlussstück erschwert und/oder verhindert wird, wobei das Erschweren und/oder Verhindern durch zumindest teilweises Überdecken und/oder zumindest teilweises Umschließen der zur reversiblen Sicherung verwendeten Sicherungsmittel und/oder des Anschlussstücks und/oder des Gegenstücks und/oder durch Erschweren und/oder Verhindern des Zugriffs auf die zur reversiblen Sicherung verwendeten Sicherungsmittel und/oder Erschweren und/oder Verhindern des Bedienens und/oder Lösens der zur reversiblen Sicherung verwendeten Sicherungsmittel erfolgt, wobei das Erschweren und/oder Verhindern insbesondere durch das Aufschieben der Überwurfhülse und/oder die dadurch erreichte Lage und/oder Anordnung der Überwurfhülse, insbesondere relativ zum Anschussstück, Gegenstück und/oder Sicherungsmittel, bewirkt wird.

Dabei erfolgen das Aufschieben und das unlösbare Verrasten durch Verschieben der Überwurfhülse auf dem Anschlussstück und/oder einem Verschiebebereich und/oder einem daran angrenzenden Schlauch und/oder Rohr und/oder durch Verschieben der Überwurfhülse auf dem Anschlussstück und/oder durch Aufschieben einer Überwurfhülse auf das Anschlussstück.

Durch das Aufschieben der Überwurfhülse insbesondere auf das und/oder auf dem Anschlussstück und das unlösbare Verrasten der Überwurfhülse mit dem Anschlussstück wird das Lösen der reversiblen Sicherung erschwert und/oder verhindert und dadurch die reversibel gesicherte Verbindung zu einer nicht entkonnektierbaren verändert.

Vorteilhafterweise erfolgt das Aufschieben durch Bewegen, insbesondere Verschieben, der Überwurfhülse auf einem/dem und/oder entlang eines/des Verschiebebereich(s), wobei der Verschiebebereich eine Längserstreckung, insbesondere eine Längserstreckung parallel zur Längserstreckung des Anschlussstücks und/oder Überwurfhülse, von mindestens 1 cm, insbesondere mindestens 5 cm, insbesondere mindestens 10 cm aufweist, und/oder erfolgt das Aufschieben durch Bewegen, insbesondere Verschieben, der Überwurfhülse um mindestens 1 cm, insbesondere mindestens 5 cm, insbesondere mindestens 10 cm, einem/dem und/oder entlang eines/des Verschiebebereich(s), wobei insbesondere während des Aufschiebens eine zusätzliche Kraft und/oder ein einer Verschiebung der Überwurfhülse relativ zum Anschlussstück entgegen wirkende Widerstand zu überwinden ist.

Mit einer solchen Länge des Verschiebebereichs bzw. einem Aufschieben der Überwurfhülse wird sichergestellt, dass sich die Anordnung bzw. der Konnektor einfach zusammenstellen lässt und insbesondere die Überwurfhülse derart aus einem Bereich entfernt von dem Konnektor auf diesen schieben lässt, dass vor Aufschieben, die Anordnung, bzw. der Konnektor einfach zusammenstellbar ist.

Vorteilhafterweise besteht der einer Verschiebung der Überwurfhülse relativ zum Anschlussstück entgegen wirkende Widerstand auf einer Länge von mindestens 1 cm, insbesondere mindestens 5 cm, insbesondere mindestens 10 cm, auf dem Verschiebebereich und/oder auf mindestens 10%, insbesondere mindestens 50%, der Länge des Verschiebebereichs besteht.

Vorteilhafterweise ist und/oder wird die Überwurfhülse zunächst auf dem Schlauch und/oder dem Rohr, welches an das Anschlussstück anschließt, angeordnet und wird die Überwurfhülse mithilfe einer, insbesondere mithilfe einer parallel zur Längserstreckung der Überwurfhülse, insbesondere zusätzlich zur Gewichtskraft der Überwurfhülse, aufgewendeten Kraft, insbesondere externen Kraft, insbesondere aufgebracht durch eine menschliche Hand, insbesondere aufgebracht an der Überwurfhülse, insbesondere relativ zum Anschlussstück, auf dem Schlauch und/oder dem Rohr und/oder auf einem Abschnitt des Anschlussstücks und/oder dem Verschiebebereich verschoben. Insbesondere ist die dabei aufgewendete Kraft größer als die Gewichtskraft der Überwurfhülse, insbesondere beträgt sie mindestens das 1,1 fache der Gewichtskraft der Überwurfhülse, insbesondere ist sie um ein Vielfaches größer als die Gewichtskraft der Überwurfhülse. Vorteilhafterweise wird die Überwurfhülse aufgrund einer aufgewendeten Kraft von weniger als 5 N und/oder mehr als 0,01 N, insbesondere weniger als 1 N, insbesondere mehr als 0,1 N, insbesondere zwischen 0,12 und 0,81 N, verschoben. Dabei wird insbesondere eine Kraft von maximal 60 N und/oder wenigstens 27,5 N, insbesondere von 35-55 N, insbesondere von 36-52 N, aufgewendet, um die Überwurfhülse mit dem Anschlussstück zu verrasten. Durch das Verrasten wird insbesondere, insbesondere durch eine entsprechende Ausbildung des Konnektors, ein Verschieben der Überwurfhülse relativ zum Anschlussstück in alle Richtungen begrenzt.

Durch eine solche zusätzliche Kraft, die benötigt und/oder aufgebracht wird, um die Überwurfhülse auf dem Verschiebebereich zu verschieben, um die Überwurfhülse mit dem Anschlussstück zu verrasten, wird besonders vorteilhafterweise ein unbeabsichtigtes Verrasten der Überwurfhülse mit dem Anschlussstück verhindert, wobei während des Aufbaus der Infusionsanordnung und nach einer Kontrolle der Anordnung durch das Verrasten der Überwurfhülse mit dem Anschlussstück ein Lösen der revisiblen Sicherung zwischen Anschlussstück und Gegenstück verhindert bzw. erschwert wird.

In Bezug auf die Eigenschaften der Verbindung, des unlösbaren Verrastens und des Konnektors und des Gegenstücks gilt vorteilhafterweise oben zum System und/oder Konnektor als vorteilhaft Ausgeführtes. Es lässt sich insbesondere durch entsprechende Ausgestaltung und/oder Auswahl des verwendeten Anschlussstücks, Gegenstücks, Sicherungsmittels, Rastmittels und/oder Konnektors verfahrensgemäß umsetzten.

Weitere vorteilhafte Merkmale sollen rein exemplarisch und nicht beschränkend nachfolgend anhand der rein schematischen Figuren erläutert werden. Dabei zeigen:
Fig.1: einen Längsschnitt durch einen Konnektor mit einem erfindungsgemäßen Anschlussstück mit einer auf dem Schlauch verschiebbar angeordneten und unverrasteten Überwurfhülse;
Fig.2: einen Längsschnitt durch den Konnektor aus Figur 1 in einem mit einem Gegenstück verbundenen Zustand ;
Fig. 3: einen Längsschnitt der Anordnung aus Fig. 2 jedoch mit auf dem Anschlussstück verrasteter Überwurfhülse und
Fig. 4: eine Aufsicht auf die Überwurfhülse und das Anschlussstück aus Fig. 3 im verrasteten Zustand der Überwurfhülse mit dem Anschlussstück.
Fig. 5: ein Schnitt durch die Überwurfhülse und das Anschlussstück aus Fig. 3 im verrasteten Zustand der Überwurfhülse mit dem Anschlussstück.

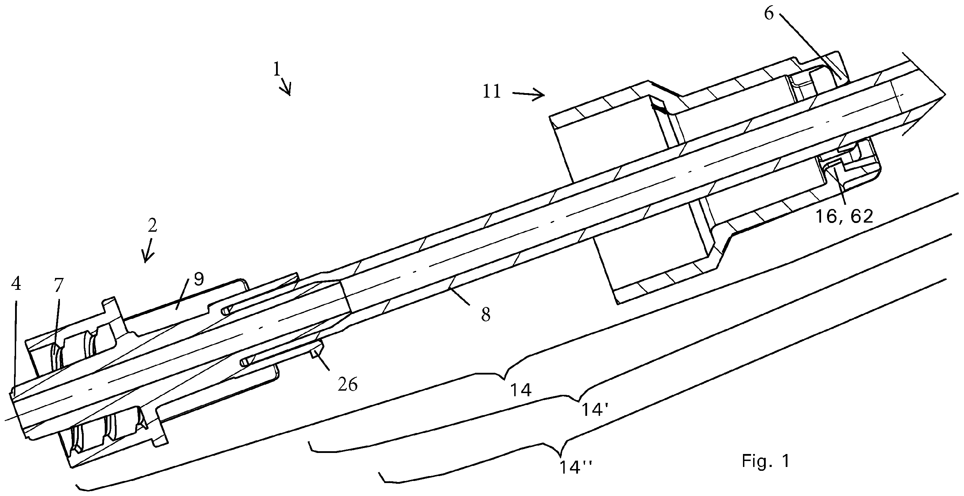

Figur 1 zeigt einen Längsschnitt eines Luer-Lock-Konnektor 1, der ein erfindungsgemäßes Anschlussstück 2 aufweist. An diesem Anschlussstück 2 ist an einem proximalen Ende, welches einem in dem Anschlussstück 2 angeordneten Luer-Gewinde 7 gegenüberliegt, ein Schlauch 8 angeordnet. Zudem weist das Anschlussstück 2 an dem distalen Ende einen Luer-Konus 4 auf, der vom Luer-Gewinde 7 zumindest teilweise umschlossen wird. Es ist ersichtlich, dass in dieser Ausführungsform der Schlauch 8, auf einen Abschnitt des Anschlussstück 2 aufgeschoben und dadurch gedehnt ist und somit einen größeren Durchmesser aufweist als in seinem weiterführenden Verlauf weg vom Anschlussstück 2. Dies ist jedoch nicht erforderlich. Am proximalen Ende des Anschlussstücks sind Rastmittel 26 angebracht, die nach außen zeigen.

Ebenso gezeigt ist eine erfindungsgemäße Überwurfhülse 11, die auf dem Schlauch 8, im Normalfall werkseitig, angeordnet ist. Diese Überwurfhülse 11 ist mit Rastmitteln 62 versehen, die als Gegenrastmitteln 62 zu den Rastmittel 26 des Anschlussstücks 2 zu verstehen sind.

Zudem weißt die Überwurfhülse 11 Eingriffsmittel 6 auf. Mit diesen Eingriffsmitteln 6 wird ein Verschieben der auf dem Schlauch 8 angeordneten Überwurfhülse 11 auf einem Verschiebebereich 14 erschwert, wobei der Verschiebebereich 14 den Bereich umfasst auf dem die Überwurfhülse 11 sich relativ zum Anschlussstück 2 verschieben lässt. Dabei sind die Eingriffsmittel 6 so eingerichtet, dass sie eine solche Öffnungsweite ausbilden, dass die Eingriffsmittel 6 mit einem Untermaß auf diesem Verschiebebereich 14 anliegen, eine komprimierende Kraft auf den Schlauch 8 ausüben und somit ein Verschieben der Überwurfhülse 11 relativ zum Anschlussstück 2 nur mithilfe einer zusätzlichen Kraft möglich ist. Ein unabsichtliches Verschieben und/oder Verrutschen der Überwurfhülse 11 wird somit erschwert bzw. verhindert. Die Gewichtskraft der Überwurfhülse 11 allein reicht dabei nicht aus, dass die Überwurfhülse 11 sich auf dem Verschiebebereich 14 bewegt.

Ein Verschieben auf den den gedehnten Bereich des Schlauchs 8 erfordert eine weitere Steigerung der Kraft und gewährleistet Schutz vor unbeabsichtigtem Verrasten der Rastmittel 26 und 62.

Die Eingriffsmittel 6 sind in Figur 1 distal zu den Rastmitteln 62 angeordnet, wobei der Abstand zwischen Eingriffsmittel 6 und Rastmittel 62 so eingerichtet ist, dass durch diesen Abstand ein Raum innerhalb der Überwurfhülse geschaffen wird, der mindestens der Breite der Rastmittel 26 des Anschlussstücks 2 parallel zur Längserstreckung des Anschlussstücks 2 entspricht. Die Breite der in Figur 1 dargestellten Rastmittel 26 entspricht 1 mm, und entsprechend ist die Länge des Raumes innerhalb der Überwurfhülse, definiert durch den Abstand zwischen Eingriffsmittel 6 und Rastmittel 62, 1,5 mm.

Diese Rastmittel 26 des Anschlussstücks 2 weisen insofern eine vorteilhafte Ausbildung in Form einer Trapezform auf, als dass eine am proximalen Ende des Anschlussstücks 2 liegenden erste Kante gebrochen ist, was ein Aufschieben der Überwurfhülse 11 mit ihren Rastmitteln 62 vereinfacht.

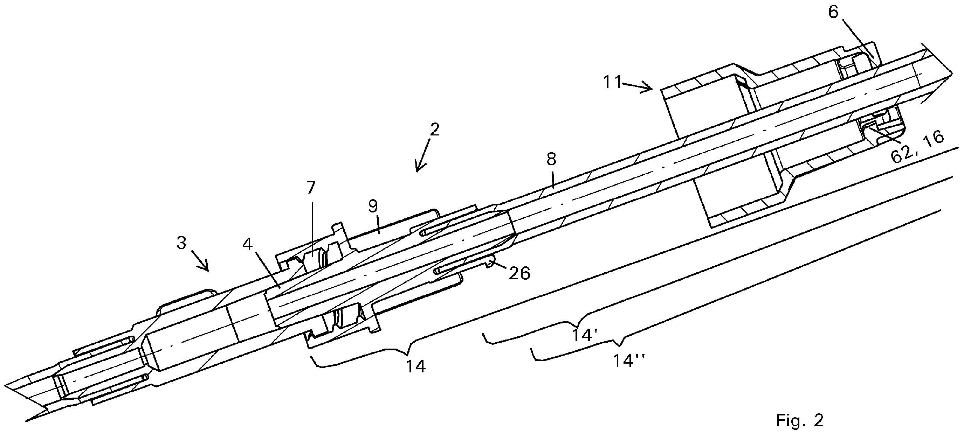

Figur 2 zeigt in einem Längsschnitt durch den Konnektor 1 der Figur 1, ein zu dem Anschlussstück 2 passendes Gegenstück 3 und einem zum Gewinde 7 passendes Gegengewinde 17 im verbundenen Zustand und durch Verschrauben des Gewindes 3 mit dem Gegengewinde 17 teilweise gesichert. In dieser Darstellung ist das Gewinde nicht vollständig genutzt. Auf Grund von Fertigungstoleranzen ist die Weite der Einführung des Konus 4 in des Gegenstück 3 sehr unterschiedlich, sodass das in der Praxis unterschiedlich viele der Gewindezüge zur Sicherung genutzt werden. Zudem zeigt die Figur 2, wie auch die Figur 1, ein in dem Anschlussstück 2 vorgesehenes vorteilhaftes Zwischenstück 9 auf, welches durch entsprechende Grifffortsätze das Verschrauben des Anschlussstücks mit einem Gegenstück vereinfacht. Veranschaulicht sind auch der Bereich 14', auf dem die Rastmittel 62 der Überwurfhülse 11 verschoben werden können, wobei diese in der ganz rechten Position in der Figur bereits mit den Rastmitteln 26 des Anschlussstücks 2 verrastet sind, und der Bereich 14", auf dem die Eingriffsmittel 6 der Überwurfhülse verschoben werden können, wobei die Rastmittel 62 in der ganz rechten Position des Bereichs 14" der Eingriffsmittel 6 in der Figur bereits mit den Rastmitteln 26 des Anschlussstücks 2 verrastet sind.

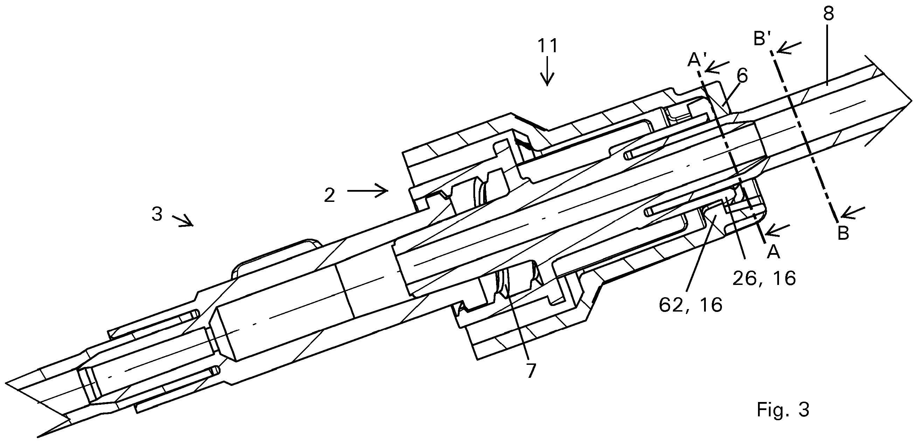

In der Figur 3 ist wieder ein Längsschnitt durch die Elemente aus Figur 2 dargestellt, wobei Gegenstück 3 und Anschlussstück 2 durch weiteres Verschrauben vollständig verbunden und durch die Verschraubung gesichert sind. Dazu ist die Überwurfhülse 11 mit dem Anschlussstück 2 verrastet und die Anordnung dadurch in einen nicht-entkonnektierbaren Zustand überführt.

In diesem Zustand ist die Überwurfhülse 11 mit dem Anschlussstück 2 mittels der Rastmittel 26 des Anschlussstücks 2 und der Rastmittel 62 der Überwurfhülse 11 unlösbar verrastet. In einem solchen Zustand ist ein Verschieben der Überwurfhülse 11 relativ zum Anschlussstück 2 in alle Richtungen eng begrenzt. Dabei sind die Rastmittel 16 so eingerichtet, dass die Überwurfhülse 11 relativ zum Anschlussstück 2 frei drehen kann, wodurch eine weitere Beeinflussung der Verschraubung des Gewindes 7 mit dem Gegengewinde 17 ohne Zerstörung der Überwurfhülse 11 ohne weitere Hilfsmittel nicht möglich ist.

Die Längserstreckung der Überwurfhülse 11 wurde dabei so ausgewählt, dass in dem verrasteten Zustand der Überwurfhülse 11 mit dem Anschlussstück 2 das Gewinde 7 des Anschlussstücks 2 und auch das Anschlussstück 2 zumindest so verdeckt sind, dass ein zerstörungsfreier Zugriff auf das Anschlussstück 2 zum Lösen der Verschraubung ohne Werkzeug nicht möglich ist. Der nicht durch die Überwurfhülse 11 verdeckte Bereich des Anschlussstücks 2 ist dabei so klein, dass zumindest durch menschliche Hände die Verschraubung nicht gelöst werden kann.

Das gezeigte Anschlussstück 2 weist an seinem Abschnitt, der den Schlauch 8 aufnimmt, einen größeren Außendurchmesser auf als der Innendurchmesser des Schlauchs 8. Der Schlauch 8 wird daher an diesem Abschnitt gedehnt, wodurch der Schlauch 8 und somit der Verschiebebereich 14 zumindest an diesem Abschnitt einen gegenüber dem restlichen Schlauch 8 erweiterten Außendurchmesser aufweist. Dies ist aber nicht erforderlich. Um in den verrasteten Zustand der Überwurfhülse 11 mit dem Anschlussstück 2 zu gelangen, muss die Überwurfhülse 11 auf dem Verschiebebereich 14 auf das Anschlussstück 2 mit einer grö-ßeren Kraft verschoben werden, als zum Verschieben auf dem restlichen Schlauch 8 erforderlich ist, da unter anderem die Eingriffsmittel 6 auf einen Abschnitt des Verschiebebereichs 14 mit erweitertem Außendurchmesser verschoben werden müssen. Die größere Kraft ist dabei größer als die zusätzlich benötigte Kraft, um die Überwurfhülse 11 auf Schlauch 8 des Verschiebebereichs 14 im zu verschieben.

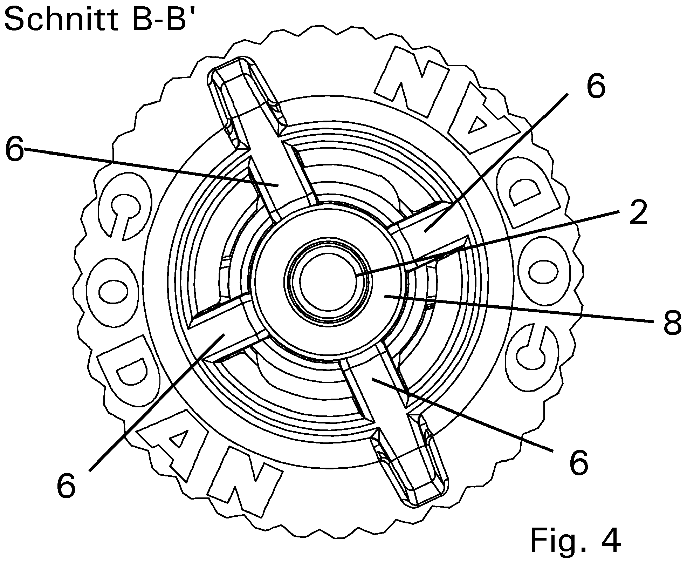

In der Figur 4 ist eine Aufsicht auf die Anordnung aus der Figur 3 von der Linie B-B' dargestellt, wobei aus einer Perspektive auf diese Anordnung dargestellt ist, die auf das Ende der Überwurfhülse 11 aufsieht, welches nicht das Gewinde 7 des Anschlussstücks 2 zumindest teilweise verdeckt. In einer solchen Aufsicht sind die Eingriffsmittel 6 der Überwurfhülse 11 gezeigt, die an dem Verschiebebereich 14, hier dem Schlauch 8, anliegen. In dieser Ausführungsform der Überwurfhülse 11sind vier Stege als Eingriffsmittel 6 an der Überwurfhülse 11 angeordnet, wobei die Stege so angeordnet sind, das Ringsegemente zwischen den Stegen gleiche Abstände bilden, und somit die Stege symmetrisch in einem Kreis um den Verschiebbereich 14 angeordnet sind. Aufgrund des verrasteten Zustandes der Überwurfhülse 11 mit dem Anschlussstück 2 liegen die Eingriffsmittel 6 auf einem Abschnitt des Schlauchs 8, der aufgrund der Aufnahme des Schlauchs 8 auf dem Anschlussstück 2 einen vergrößerten Außendurchmesser aufweist.

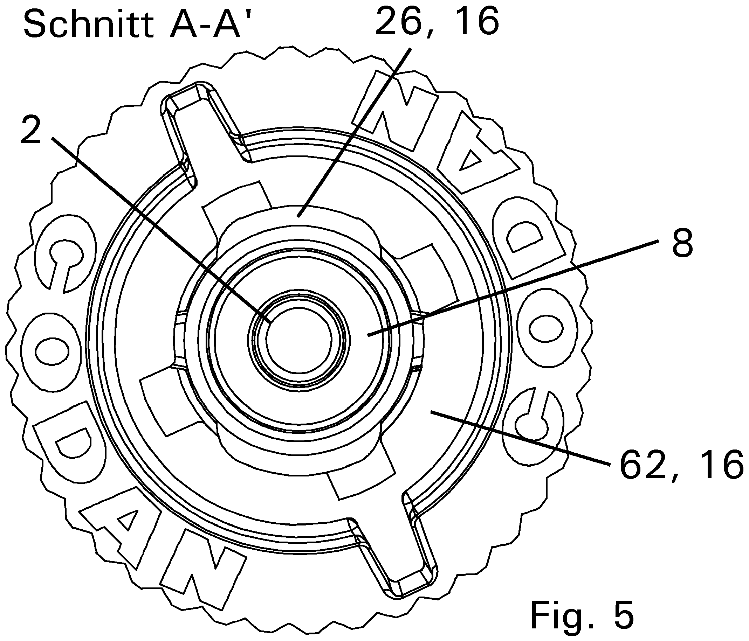

In der Figur 5 ist ein Schnitt auf Höhe der Linie A-A' durch die Anordnung aus der Figur 3 dargestellt, wobei der Konnektor 1 ausgehend vom Ende der Überwurfhülse 11, welches nicht das Gewinde 7 des Anschlussstücks 2 zumindest teilweise verdeckt, betrachtet dargestellt ist.

Der verrastete Zustand der Überwurfhülse 11 mit dem Anschlussstück 2 ist insofern gezeigt, dass die Rastmittel 26 des Anschlussstücks 2 auf über den Rastmitteln 62 liegen. Der Rastmittelaußendurchmesser der Rastmittel 26 des Anschlussstücks 2 ist größer ausgebildet als der Durchmesser der Rastmittelöffnungsweite des Rastmittels 62 der Überwurfhülse 11. In dieser Aufsicht ist ebenso gezeigt, dass die Rastmittel 26 des Anschlussstücks 2 mit den Rastmitteln 62 der Überwurfhülse 11 zusammen einen fast vollständigen Kreis ergeben. Ein Lösen durch Drehen ist nicht möglich. Somit ist das Anschlussstück 2 unlösbar verrastet mit der Überwurfhülse 11.

### Bezugszeichenliste:

- 1: Konnektor
- 2: Anschlussstück
- 3: Gegenstück
- 4: Konus
- 6: Eingriffsmittel
- 7: Gewinde
- 8: Schlauch
- 9: Zwischenstück
- 11: Überwurfhülse
- 14: Verschiebebereich
- 14': Bereich auf dem die Rastmittel 62 der Überwurfhülse verschoben werden können
- 14": Bereich auf dem die Eingriffsmittel 6 verschoben werden können
- 16: Rastmittel
- 17: Gegengewinde
- 26: Rastmittel des Anschlussstücks
- 62: Rastmittel der Überwurfhülse

## Patentansprüche

1. Konnektor (1) und/oder Adapter (1) mit
(i) einem Anschlussstück (2) zum Verbinden mit
einem passenden Gegenstück (3) zwecks Substanztransport (3)
(ii) wobei das Anschlussstück (2)
(a) einen Konus (4) und
(b) ein Sicherungsmittel (7) zum reversiblen Sichern einer Verbindung mit dem passenden Gegenstück aufweist, und
(iii) einem Schlauch (8), und
(iv) einem Verschiebebereich (14) und
(v) einer Überwurfhülse (11), die eingerichtet ist,
(a) zumindest teilweise um das Anschlussstück (2) und/oder auf dem Verschiebebereich (14) drehbar zu sein, und
(vi) wobei an dem Konnektor (1) und/oder an der Überwurfhülse (11) mindestens ein Rastmittel (16,26,62) vorgesehen ist, zum unlösbaren Verrasten des Anschlussstücks (2) mit der Überwurfhülse (11), wobei der Konnektor so ausgebildet ist, dass in einem unlösbar verrasteten Zustand des Anschlussstücks (2) mit der Überwurfhülse ein Verschieben der Überwurfhülse (11) relativ zum Anschlussstück (2) in alle Richtungen begrenzt ist,
**dadurch gekennzeichnet, dass**
(i) die Überwurfhülse (11) und der Verschiebebereich (14) so ausgebildet sind, dass
(a) die Überwurfhülse (11) mindestens ein Eingriffsmittel (6) aufweist,
(i) wobei das mindestens eine Eingriffsmittel (6) eingerichtet ist, mit dem Verschiebebereich (14) zumindest teilweise so verbunden zu sein, den Verschiebebereich (14) zumindest teilweise so zu umschließen, dass
das mindestens eine Eingriffsmittel (6) eine Öffnung definiert, die kleiner ist als eine maximale Erstreckung des Querschnitts durch den Verschiebebereich (14) senkrecht zu einer Längserstreckung des Verschiebebereichs (14), und dass
(ii) die Überwurfhülse (11) so ausgebildet ist, dass im unlösbar verrasteten Zustand des Anschlussstücks (2) mit der Überwurfhülse
(a) das Bedienen und/oder Lösen des Sicherungsmittels durch zumindest teilweises Verdecken des Sicherungsmittels und/oder des Gegenstückes und/oder des Konnektors erschwert und/oder verhindert.

2. Konnektor nach Anspruch 1 **dadurch gekennzeichnet, dass** zumindest im unlösbar verrasteten Zustand der Überwurfhülse (11) mit dem Anschlussstück die Überwurfhülse (11) um den Konnektor (1) drehbar ist.

3. Konnektor nach einem der vorherigen Ansprüche, **dadurch gekennzeichnet, dass** durch das durch die Überwurfhülse (11) im unlösbar verasteten Zustand bewirkte zumindest teilweise Umschließen des Sicherungsmittels und/oder Konnektors und/oder Gegenstücks ein Lösen der reversiblen Sicherung erschwert.

4. Konnektor nach einem der vorherigen Ansprüche, **dadurch gekennzeichnet, dass** die durch die Eingriffsmittel (6) gebildete Öffnungsweite einen kleineren Durchmesser als der maximale äußere Durchmesser des Verschiebebereichs (14) aufweist.

5. Konnektor nach einem der vorherigen Ansprüche, **dadurch gekennzeichnet, dass** die Überwurfhülse (11) mindestens ein erstes Rastmittel (62) aufweist, das eine Rastmittelöffnungsweite ausbildet, und dass das Anschlussstück mindestens ein zweites Rastmittel (26) aufweist, das einen Rastmittelaußendurchmesser ausbildet, wobei der Durchmesser der Rastmittelöffnungsweite weniger als 8 mm beträgt und/oder kleiner ist als der Rastmittelaußendurchmesser,
und/oder
die Rastmittel (26) des Anschlussstücks (2) mit ihren äußeren Erstreckungen einen Kreis definieren, der einen Außendurchmesser aufweist, der kleiner ist als der Innendurchmesser eines Abschnitts der Überwurfhülse (11), in und/oder an welchem die Rastmittel des Anschlussstücks (2) im unlösbar verrasteten Zustand des Anschlussstücks (2) mit der Überwurfhülse (11) aufgenommen werden und/oder anliegen.

6. Konnektor nach einem der vorherigen Ansprüche, **dadurch gekennzeichnet, dass** das Eingriffsmittel (6) mindestens einen Steg aufweist.

7. Konnektor nach einem der vorherigen Ansprüche, **dadurch gekennzeichnet, dass** die Rastmittel (16,26,62) derart ausgeformt sind, dass die Rastmittel (16,26,62) in ihrer Querschnittsebene parallel zur Längserstreckung des Konnektors (1) und/oder der Überwurfhülse (11) eine Trapezform aufweisen.

8. Konnektor nach einem der vorherigen Ansprüche, **dadurch gekennzeichnet, dass** die Rastmittel (62,26) der Überwurfhülse (11) und/oder des Anschlussstücks (2), insbesondere im verrasteten Zustand des Anschlussstücks mit der Überwurfhülse, einen fast vollständigen Ring ausbilden.

9. Konnektor nach einem der vorherigen Ansprüche, **dadurch gekennzeichnet, dass**
die Rastmittel (26,62) des Anschlussstücks (2) und/oder der Überwurfhülse (11) in einer Aufsicht auf die Rastmittel (16,26,62), die parallel zur Längserstreckung des Anschlussstücks (2) und/oder der Überwurfhülse (11) ist, jeweils ein Segment eines Rings ausbilden, wobei die längste Sehne durch jedes Segment des Rastmittels (26) des Anschlussstücks (2) größer ist als der Abstand zwischen den Rastmitteln (62) der Überwurfhülse (11), den die Rastmittel (62) der Überwurfhülse (11) in einer Aufsicht auf die Rastmittel (16), die parallel zur Längserstreckung des Anschlussstücks (2) und/oder der Überwurfhülse (11) ist, aufweisen
und/oder
die Rastmittel (26,62) des Anschlussstücks (2) und/oder der Überwurfhülse (11) in einer Aufsicht auf die Rastmittel (26,62), die parallel zur Längserstreckung des Anschlussstücks (2) und/oder der Überwurfhülse (11) ist, jeweils ein Segment eines Rings ausbilden, wobei die längste Sehne durch jedes Segment des Rastmittels (26) des Anschlussstücks (2) größer ist als der Abstand zwischen den Rastmitteln (26) des Anschlussstücks (2), den die Rastmittel (26) des Anschlussstücks (2) in einer Aufsicht auf die Rastmittel (16), die parallel zur Längserstreckung des Anschlussstücks (2) und/oder der Überwurfhülse (11) ist, aufweisen
und/oder
die Rastmittel (26,62) des Anschlussstücks (2) und/oder der Überwurfhülse (11) in einer Aufsicht auf die Rastmittel (26,62), die parallel zur Längserstreckung des Anschlussstücks (2) und/oder der Überwurfhülse (11) ist, jeweils ein Segment eines Rings ausbilden, wobei ein erstes Rastmittel (26) des Anschlussstücks (2) zumindest teilweise von einem Rastmittels (62) der Überwurfhülse (11) verdeckt wird.

10. Konnektor nach einem der vorherigen Ansprüche, **dadurch gekennzeichnet, dass** das Sicherungsmittel (7) fest und/oder steif mit dem Konus (4) und/oder mit dem Anschlussstück (2) verbunden ist.

11. Konnektor nach einem der vorherigen Ansprüche, **dadurch gekennzeichnet, dass** die Eingriffsmittel der Überwurfhülse (6) an der Überwurfhülse (11) distal und/oder am von dem Sicherungsmittel entfernten Ende der Überwurfhülse (7) ausgebildet sind und/oder dass die Überwurfhülse so positioniert ist, dass die Eingriffsmittel der Überwurfhülse am vom Konus entfernten Ende des Anschlussstücks (2) und/oder am vom Konus entfernten Ende des Verschiebbereichs (14) auf dem Verschiebebereich angeordnet sind.

12. System aufweisend mindestens einen Konnektor (1) mit einem Anschlussstück (2) nach einem der vorherigen Ansprüche und mindestens ein Gegenstück (3).

13. Verfahren zur Erstellung einer Infusionsanordnung zum Transport unter Verbindung eines Anschlussstücks mit einem Gegenstück, unter Verwendung eines Systems nach Anspruch 12 und/oder eines Konnektors nach einem der Ansprüche 1 bis 11, wobei
(i) das Anschlussstück (2) mit dem Gegenstück (3) reversible verbunden wird und diese Verbindung reversibel gesichert wird, **dadurch gekennzeichnet, dass**
(ii) das Lösen der reversiblen Sicherung durch Aufschieben einer Überwurfhülse (11) und unlösbares Verrasten der Überwurfhülse (11) mit dem Anschlussstück (2) erschwert und/oder verhindert wird, wobei das Erschweren und/oder Verhindern durch zumindest teilweises Überdecken und/oder zumindest teilweises Umschließen der zur reversiblen Sicherung verwendeten Sicherungsmittel und/oder des Anschlussstücks und/oder des Gegenstücks und/oder durch Erschweren und/oder Verhindern des Zugriffs auf die zur reversiblen Sicherung verwendeten Sicherungsmittel und/oder Erschweren und/oder Verhindern des Bedienens und/oder Lösens der zur reversiblen Sicherung verwendeten Sicherungsmittel erfolgt,
(iii) wobei das Aufschieben und das unlösbare Verrasten durch Verschieben der Überwurfhülse (11) auf dem Anschlussstück (2) und/oder Verschiebebereich und/oder einem daran angrenzenden Schlauch (5) und/oder Rohr und/oder durch Verschieben der Überwurfhülse (11) auf dem Anschlussstück (2) und/oder durch Aufschieben einer Überwurfhülse (11) auf das Anschlussstück.

## Claims

1. A connector (1) and/or adapter (1) with
(i) a connecting piece (2) for connection to a suitable counterpart (3) for the purpose of substance transport (3)
(ii) whereby the connecting piece (2) contains
(a) a cone {4} and
(b) a securing means (7) for reversibly securing a connection to the mating component, and
(iii) a hose (8), and
(iv) a displacement range (14) and
(v) a sleeve (11), which is fitted,
(a) to be at least partially rotatable about the connecting piece (2) and/or on the displacement area (14), and
(vi)wherein at least one latching means (16, 26, 62) is provided on the connector (1) and/or on the sleeve (11) for the non-detachable latching of the connecting piece (2) with the sleeve (11), wherein the connector is designed such that in a non-detachably latched state of the connecting piece (2) with the sleeve a displacement of the sleeve (11) relative to the connecting piece (2) is limited in all directions,
**characterised in that**
(i) the sleeve (11) and the displacement area (14) are designed such that
(a) the sleeve (11) has at least one engagement means (6),
(i) wherein said at least one engagement means (6) is arranged to be at least partially connected to the displacement region (14) so as to at least partially enclose the displacement region (14) such that
said at least one engagement means (6) defines an opening which is smaller than a maximum extension of the cross-section through the displacement region (14) perpendicular to a longitudinal extension of the displacement region (14), and **in that**
(ii) the sleeve (11) is designed in such a way that in the non-detachably latched state of the connecting piece (2) with the sleeve
(a) makes it difficult and/or prevents the operation and/or release of the securing means by at least partially covering the securing means and/or the counterpart and/or the connector.

2. A connector according to claim 1, **characterised in that** the sleeve (11) can be rotated around the connector (1) at least when the sleeve (11) is in the non-detachably latched state with the connecting piece.

3. A connector according to one of the preceding claims, **characterised in that** said at least partial enclosure of the securing means and/or connector and/or counterpart caused by the sleeve (11) in the non-detachably latched state makes it more difficult to release the reversible securing means.

4. A connector according to one of the preceding claims, **characterised in that** the opening width formed by the engagement means (6) has a smaller diameter than the maximum outer diameter of the displacement region (14).

5. A connector according to one of the preceding claims, **characterised in that** the sleeve (11) has at least one first detent means (62) which forms a detent means opening width, and **in that** the connector has at least one second detent means (26) which forms a detent means outer diameter, the diameter of the detent means opening width being less than 8 mm and/or being smaller than the detent means outer diameter, and/or
the latching means (26) of the connecting piece (2) define with their outer extensions a circle which has an outer diameter which is smaller than the inner diameter of a section of the sleeve (11), in and/or against which the latching means of the connecting piece (2) are received and/or bear in the non-detachably latched state of the connecting piece (2) with the sleeve (11).

6. A connector according to one of the preceding claims, **characterised in that** the engagement means (6) has at least one web.

7. A connector according to one of the preceding claims, **characterised in that** the latching means (16, 26, 62) are shaped in such a way that the latching means (16, 26, 62) have a trapezoidal shape in their cross-sectional plane parallel to the longitudinal extent of the connector (1) and/or the sleeve (11).

8. A connector according to one of the preceding claims, **characterised in that** the latching means (62, 26) of the sleeve (11) and/or of the connecting piece (2) form an almost complete ring, in particular in the latched state of the connecting piece with the sleeve.

9. A connector according to one of the preceding claims, **characterised in that**
the latching means (26, 62) of the connecting piece (2) and/or of the sleeve (11) each form a segment of a ring in a view of the latching means (1 6, 26, 62) which is parallel to the longitudinal extent of the connecting piece (2) and/or of the sleeve (11), wherein the longest chord through each segment of the detent means (26) of the connecting piece (2) is greater than the distance between the detent means (62) of the sleeve (11) which the detent means (62) of the sleeve (11) have in a top view of the detent means (16), which is parallel to the longitudinal extent of the connecting piece (2) and/or the sleeve (11), and/or
the latching means (26, 62) of the connecting piece (2) and/or of the sleeve (11) each form a segment of a ring in a view of the latching means (26, 62) which is parallel to the longitudinal extension of the connecting piece (2) and/or of the sleeve (11), wherein the longest chord through each segment of the detent means (26) of the connecting piece (2) is greater than the distance between the detent means (26) of the connecting piece (2) which the detent means (26) of the connecting piece (2) have in a view of the detent means (16) which is parallel to the longitudinal extent of the connecting piece (2) and/or the sleeve (11), and/or
the latching means (26, 62) of the connecting piece (2) and/or of the sleeve (11) each form a segment of a ring in a view of the latching means (26, 62) which is parallel to the longitudinal extent of the connecting piece (2) and/or of the sleeve (11), wherein a first latching means (26) of the connecting piece (2) is at least partially covered by a latching means (62) of the sleeve (11).

10. A connector according to one of the preceding claims, **characterised in that** the securing means (7) is firmly and/or rigidly connected to the cone (4) and/or to the connecting piece (2).

11. A connector according to one of the preceding claims, **characterised in that** the engagement means of the sleeve (6) are formed on the sleeve (11) distally and/or at the end of the sleeve (7) remote from the securing means and/or **in that** the sleeve is positioned such that the engagement means of the sleeve are arranged on the displacement region at the end of the connecting piece (2) remote from the cone and/or at the end of the displacement region (14) remote from the cone.

12. A system comprising at least one connector (1) with a connecting piece (2) according to one of the previous claims and at least one counterpart (3).

13. A method of creating an infusion assembly for transport by connecting a connector to a counterpart, using a system according to claim 12 and/or a connector according to any one of claims 1 to 11, wherein
(i) the connecting piece (2) is reversibly connected to the counterpart (3) and this connection is reversibly secured, **characterised in that**
(ii) the release of the reversible securing means is made more difficult and/or prevented by sliding on a sleeve (11) and non-detachable latching of the sleeve (111) with the connecting piece (2), wherein the impeding and/or preventing is effected by at least partially covering and/or at least partially enclosing the securing means used for the reversible securing and/or the connecting piece and/or the mating piece and/or by impeding and/or preventing access to the securing means used for the reversible securing and/or impeding and/or preventing the operation and/or release of the securing means used for the reversible securing,
(iii) wherein the sliding on and the non-detachable latching is achieved by sliding the sleeve (11) on the connecting piece (2) and/or sliding area and/or a hose (5} and/or pipe adjacent thereto and/or by sliding the sleeve (11) on the connecting piece {2) and/or by sliding a sleeve (11) onto the connecting piece.

## Revendications

1. Connecteur (1) et/ou adaptateur (1) avec
(i) un connecteur (2) permettant la connexion à une contrepartie appropriée (3) aux fins du transport de la substance (3)
(ii) la pièce de connexion (2) contient
(a) un cône {4} et
(b) un moyen de fixation (7) pour fixer de manière réversible une connexion à l'élément d'accouplement, et
(iii) un tuyau (8), et
(iv) une plage de déplacement (14) et
(v) un manchon (11), qui est mis en place,
(a) pour être au moins partiellement rotatif autour de la pièce de connexion (2) et/ou sur la zone de déplacement (14), et
(vi)dans lequel au moins un moyen de verrouillage (16, 26, 62) est prévu sur le connecteur (1) et/ou sur le manchon (11) pour le verrouillage indétachable de la pièce de connexion (2) avec le manchon (11), dans lequel le connecteur est conçu de telle sorte que dans un état de verrouillage indétachable de la pièce de connexion (2) avec le manchon, un déplacement du manchon (11) par rapport à la pièce de connexion (2) est limité dans toutes les directions,
**se caractérise par le fait que**
(i) le manchon (11) et la zone de déplacement (14) sont conçus de telle sorte que
(a) le manchon (11) comporte au moins un moyen d'engagement (6),
(i) dans lequel ledit au moins un moyen d'engagement (6) est conçu pour être au moins partiellement relié à la zone de déplacement (14) de manière à entourer au moins partiellement la zone de déplacement (14), de sorte que
ledit au moins un moyen d'engagement (6) définit une ouverture qui est plus petite qu'une extension maximale de la section transversale à travers la zone de déplacement (14) perpendiculaire à une extension longitudinale de la zone de déplacement (14), et en ce que
(ii) le manchon (11) est conçu de manière à ce que, dans l'état de verrouillage non détachable de la pièce de connexion (2) avec le manchon
(a) rend difficile et/ou empêche le fonctionnement et/ou la libération du moyen de fixation en recouvrant au moins partiellement le moyen de fixation et/ou la contrepartie et/ou le connecteur.

2. Connecteur selon la revendication 1, **caractérisé par le fait que** le manchon (11) peut être tourné autour du connecteur (1) au moins lorsque le manchon (11) est dans l'état de verrouillage non détachable avec la pièce de connexion.

3. Connecteur selon l'une des revendications précédentes, **caractérisé en ce que** ladite fermeture au moins partielle du moyen de sécurisation et/ou du connecteur et/ou de la contrepartie causée par le manchon (11) dans l'état de verrouillage non détachable rend plus difficile la libération du moyen de sécurisation réversible.

4. Connecteur selon l'une des revendications précédentes, **caractérisé par le fait que** la largeur d'ouverture formée par les moyens d'engagement (6) a un diamètre inférieur au diamètre extérieur maximal de la zone de déplacement (14).

5. Connecteur selon l'une des revendications précédentes, **caractérisé en ce que** le manchon (11) comporte au moins un premier moyen d'encliquetage (62) qui forme une largeur d'ouverture du moyen d'encliquetage, et **en ce que** le connecteur comporte au moins un second moyen d'encliquetage (26) qui forme un diamètre extérieur du moyen d'encliquetage, le diamètre de la largeur d'ouverture du moyen d'encliquetage étant inférieur à 8 mm et/ou étant plus petit que le diamètre extérieur du moyen d'encliquetage, et/ou
les moyens de verrouillage (26) de la pièce de connexion (2) définissent avec leurs extensions extérieures un cercle dont le diamètre extérieur est inférieur au diamètre intérieur d'une section du manchon (11), dans lequel et/ou contre lequel les moyens de verrouillage de la pièce de connexion (2) sont reçus et/ou portent dans l'état verrouillé non détachable de la pièce de connexion (2) avec le manchon (11).

6. Connecteur selon l'une des revendications précédentes, **caractérisé par le fait que** le moyen d'engagement (6) comporte au moins une âme.

7. Connecteur selon l'une des revendications précédentes, **caractérisé en ce que** les moyens de verrouillage (16, 26, 62) sont façonnés de telle sorte que les moyens de verrouillage (16, 26, 62) ont une forme trapézoïdale dans leur plan de section transversale parallèle à l'étendue longitudinale du connecteur (1) et/ou du manchon (11).

8. Connecteur selon l'une des revendications précédentes, **caractérisé en ce que** les moyens de verrouillage (62, 26) du manchon (11) et/ou de la pièce de connexion (2) forment un anneau presque complet, en particulier à l'état verrouillé de la pièce de connexion avec le manchon.

9. Connecteur selon l'une des revendications précédentes, **caractérisé par le fait que** les moyens de verrouillage (26, 62) de la pièce de connexion (2) et/ou du manchon (11) forment chacun un segment d'un anneau dans une vue des moyens de verrouillage (1 6, 26, 62) qui est parallèle à l'étendue longitudinale de la pièce de connexion (2) et/ou du manchon (11), dans lequel la corde la plus longue à travers chaque segment du moyen d'encliquetage (26) de la pièce de connexion (2) est supérieure à la distance entre les moyens d'encliquetage (62) du manchon (11) que les moyens d'encliquetage (62) du manchon (11) ont dans une vue de dessus du moyen d'encliquetage (16), qui est parallèle à l'étendue longitudinale de la pièce de connexion (2) et/ou du manchon (11), et/ou
les moyens d'encliquetage (26, 62) de la pièce de connexion (2) et/ou du manchon (11) forment chacun un segment d'un anneau dans une vue des moyens d'encliquetage (26, 62) qui est parallèle à l'extension longitudinale de la pièce de connexion (2) et/ou du manchon (11), dans lequel la corde la plus longue à travers chaque segment des moyens d'arrêt (26) de la pièce de connexion (2) est supérieure à la distance entre les moyens d'arrêt (26) de la pièce de connexion (2) que les moyens d'arrêt (26) de la pièce de connexion (2) ont dans une vue des moyens d'arrêt (16) qui est parallèle à l'extension longitudinale de la pièce de connexion (2) et/ou du manchon (11), et/ou
les moyens de verrouillage (26, 62) de la pièce de connexion (2) et/ou du manchon (11) forment chacun un segment d'anneau dans une vue des moyens de verrouillage (26, 62) qui est parallèle à l'étendue longitudinale de la pièce de connexion (2) et/ou du manchon (11), dans laquelle un premier moyen de verrouillage (26) de la pièce de connexion (2) est au moins partiellement recouvert par un moyen de verrouillage (62) du manchon (11).

10. Connecteur selon l'une des revendications précédentes, **caractérisé en ce que** le moyen de fixation (7) est fermement et/ou rigidement relié au cône (4) et/ou à la pièce de connexion (2).

11. Connecteur selon l'une des revendications précédentes, **caractérisé en ce que** les moyens d'engagement du manchon (6) sont formés sur le manchon (11) distalement et/ou à l'extrémité du manchon (7) éloignée des moyens de fixation et/ou **en ce que** le manchon est positionné de telle sorte que les moyens d'engagement du manchon sont disposés sur la zone de déplacement à l'extrémité de la pièce de connexion (2) éloignée du cône et/ou à l'extrémité de la zone de déplacement (14) éloignée du cône.

12. Système comprenant au moins un connecteur (1) avec une pièce de connexion (2) selon l'une des revendications précédentes et au moins une contrepartie (3).

13. Procédé de création d'un assemblage de perfusion pour le transport en connectant un connecteur à un homologue, en utilisant un système selon la revendication 12 et/ou un connecteur selon l'une quelconque des revendications 1 à 11, dans lequel
(i) la pièce de connexion (2) est reliée de manière réversible à la pièce d'accouplement (3) et cette liaison est sécurisée de manière réversible, **caractérisée par le fait que**
(ii) la libération des moyens de fixation réversibles est rendue plus difficile et/ou empêchée par le glissement d'un manchon (11) et l'encliquetage indémontable du manchon (11) avec la pièce de connexion (2), l'empêchement et/ou la prévention étant effectués en recouvrant au moins partiellement et/ou en enfermant au moins partiellement les moyens de fixation utilisés pour la fixation réversible et/ou la pièce de liaison et/ou la pièce d'accouplement et/ou en entravant et/ou en empêchant l'accès aux moyens de fixation utilisés pour la fixation réversible et/ou en entravant et/ou en empêchant l'opération et/ou la libération des moyens de fixation utilisés pour la fixation réversible,
(iii) dans lequel le glissement et le verrouillage indémontable sont obtenus en faisant glisser le manchon (11) sur la pièce de connexion (2) et/ou la zone de glissement et/ou un tuyau (5} et/ou un tube adjacent à celle-ci et/ou en faisant glisser le manchon (111) sur la pièce de liaison {2) et/ou en faisant glisser un manchon (11) sur la pièce de liaison.
